# EUROPEAN PATENT APPLICATION

(11) **EP 1 491 549 A1**
(43) Date of publication of application: **29.12.2004**
(21) Application number: 03013457.1
(22) Date of filing: 24.06.2003
(51) Int. Cl.: C07H 7/027, C07H 1/00

(54) **Production of 2'-deoxynucleosides and 2'-deoxynucleoside precursors from 2-dehydro-3-deoxy-D-gluconate**

(71) Applicant: Evologic S.A., 91000 Evry (FR); Marliere, Philippe, 91450 Etiolles (FR)
(72) Inventor: The designation of the inventor has not yet been filed
(74) Representative: VOSSIUS & PARTNER

(57) **Abstract**

This invention relates to a process for preparing 2'-deoxynucleoside compounds or 2'-deoxynucleoside precursors using 2-dehydro-3-deoxy-D-gluconic acid (usually abbreviated as KDG) or its salts as a starting material. A variety of 2'-deoxynucleosides and their analogues are used as a starting material for synthesis or drug formulation in production of an antiviral, anticancer or antisense agent.

## Description

This invention relates to a process for preparing 2'-deoxynucleoside compounds or 2'-deoxynucleoside precursors using 2-dehydro-3-deoxy-D-gluconic acid (usually abbreviated as KDG) or its salts as a starting material. A variety of 2'-deoxynucleosides and their analogues are used as a starting material for synthesis or drug formulation in production of an antiviral, anticancer or antisense agent.

Specifically, the invention relates to a method in which KDG or a derivative of KDG is subjected to a decarboxylation step to remove the original carboxy group of KDG. In a preferred embodiment, the KDG used in the method according to the invention is enzymatically produced from D-gluconate or D-glucosaminate.

2'-deoxynucleosides and 2'-deoxynucleoside precursors including 2-deoxy-D-ribose are used as starting material for synthesis or drug formulation, for instance, in production of antiviral and anticancer agent. 2'-deoxynucleosides or derivatives thereof and 2'-deoxynucleoside precursors are also used as reagents for research, diagnosis and synthesis of therapeutic antisense molecules.

In one method of the prior art, deoxynucleosides are generated from biological materials such as testis (WO 99/49074) or yeast or fish sperm by enzymatic cleavage of DNA. This method, however, involves several disadvantages, in particular regarding difficulties of obtaining the starting material in sufficient quantity and quality.

The main production process of 2-deoxy-D-ribose currently consists in chemical hydrolysis of DNA. In this case, the deoxyribosyl moiety originates in ribonucleotide reductase activity. No synthesis of 2-deoxy-D-ribose from KDG has been yet described.

In most living cells, deoxyribonucleosides result from a "salvage pathway" of the nucleotide metabolism. The deoxyribose moiety of deoxyribonucleosides is obtained through the reduction of a ribosyl moiety into di- or triphosphate ribonucleotides catalyzed by ribonucleotide reductases. However, the deoxyribose moiety is not recycled, but is degraded into D-glyceraldehyde-3-phosphate and acetaldehyde following the reactions of central metabolism:
- deoxynucleoside is cleaved into deoxyribose-1-phosphate and nucleobase through phosphorolysis mediated by products of the genes encoding thymidine phosphorylase (deoA), purine-nucleoside phosphorylase (deoD), uridine phosphorylase (udp) or xanthosine phosphorylase (xapA).
- deoxyribose-1-phosphate is converted into deoxyribose-5-phosphate through a reaction catalyzed by deoxyribose phosphate mutase (deoB),
- which is further degraded to D-glyceraldehyde-3-phosphate and acetaldehyde through a reaction catalyzed by deoxyribose-5-phosphate aldolase (deoC).

It has been shown that the deo enzymes also catalyze in vitro the reverse anabolic reactions: Deoxyribose-5-phosphate is obtained in vitro in the presence of purified *Escherichia coli* or *Lactobacillus plantarum* deoxyribose aldolase starting from acetaldehyde and D-glyceraldehyde-3-phosphate (Rosen et al., J. Biol. Chem., 240, (1964), 1517-1524; Pricer, J. Biol. Chem., 235, (1960), 1292-1298). Deoxyribose can also be obtained with acetaldehyde and glyceraldehyde as enzyme substrates, but only with a very low yield (Barbas, J. Am. Chem. Soc. 112 (1990), 2013-2014).

The patent application WO 01/14566 describes the enzymatic synthesis of deoxynucleosides starting from deoxyribose-1-phosphate through the combined activities of three enzymes of the deo operon, i.e. deoxyribose aldolase, deoxyribomutase and phosphorylase (thymidine or purine nucleoside phosphorylase) in a one-pot reaction, using as starting substrates glyceraldehyde-3-phosphate, acetaldehyde and a nucleobase. D-glyceraldehyde-3-phosphate can be obtained from fructose-1,6-bisphosphate by an enzymatic process.

The patent application EP 1179598 describes the use of phosphorylase to catalyze the enzymatic production of deoxynucleosides starting from deoxyribose-1-phosphate and nucleobase. The yield of deoxynucleoside synthesis is improved by precipitation of phosphate.

However, methods using enzymes of the deo operon working in the reverse direction compared to their biological function show low yields, which indicates serious drawbacks for their use.

In view of the above-described ineffectiveness of the currently applied processes for producing deoxynucleosides and deoxynucleoside precursors, it is an object of the present invention to provide means and methods for the biosynthetic production of deoxynucleosides and deoxynucleoside precursors starting from cheap and commercially available compounds without being dependent on unreliable natural sources.

In particular, there is a need for alternative methods for the production of deoxynucleosides and deoxynucleoside precursors which allow efficient and economical synthesis of deoxyribonucleosides, by means of which the drawbacks of prior art processes are eliminated.

The present invention relates to a method for producing 2'-deoxynucleosides and precursors thereof starting from 2-dehydro-3-deoxy-D-gluconic acid (KDG) or its salts and comprising a decarboxylation step.

In particular, this method is useful for producing 2-deoxy-D-ribose (DRI) as well as synthetically versatile enamine derivatives of DRI as 2'-deoxynucleoside precursors.

The decarboxylation step takes place by reacting either KDG or its salts directly, or a derivative of KDG, usually to cleave the C1-C2 bond of the KDG.

In one embodiment of the invention, KDG or one of its salts undergoes (oxidative) decarboxylation leading to 2-deoxy-D-ribonic acid (DRN) or its salts, itself being further converted into 2-deoxy-D-ribose (DRI) or 2-deoxy-D-ribitol (DRL).

In another embodiment of the invention, decarboxylation takes place by reacting KDG or its salts with an amine, leading to an enamine derivative. This high energy enamine derivative can be further converted into DRI by hydrolysis.

In another embodiment of the invention, (oxidative) decarboxylation is carried out on 3-deoxy-D-gluconic acid (DGN) or its salts and/or 3-deoxy-D-mannonic acid (DMN) or its salts as derivatives of KDG, leading to DRI. Production of a mixture of DGN and DMN takes place by reduction of KDG. The decarboxylation is preferably carried out via reaction with hydrogen peroxide.

In another embodiment of the invention, (oxidative) decarboxylation is carried out on 3-deoxy-D-glucosaminic acid (DGM) or its salts and/or 3-deoxy-D-mannosaminic acid (DMM) or its salts, leading to DRI. Production of a mixture of DGM and DMM takes place from KDG by reductive amination.

Another aspect of the invention is a convenient and cost-effective method for preparing KDG or its salts to be used in the above methods. This method starts either from D-gluconate or from D-glucosaminate through the use of recombinant enzymes. The invention provides a novel nucleotide sequence encoding a polypeptide having D-gluconate dehydratase activity and a nucleotide sequence encoding a polypeptide having D-glucosaminate deaminase activity.

The starting material used for the method of the present invention is KDG, represented by formula (I) below or one of its salts, or a protected derivative thereof wherein one ore more of the hydroxyl groups at positions 4, 5 and/or 6 are protected by a protection group known in the art.

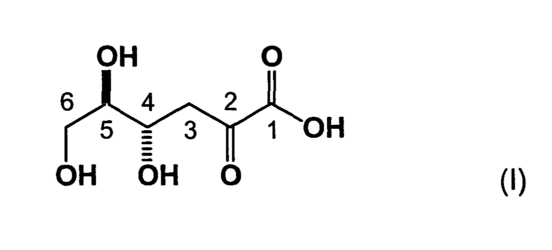

The term "2'-deoxynucleoside" as used herein relates to 2'-deoxyribonucleosides which are N-glycosides, and wherein the basic N-atom of the nucleobase or nucleobase analog is bound to the anomeric carbon atom of 2-deoxy-D-ribose, or one of its derivatives. Examples of a suitable nucleobase are adenine, cytosine, guanine, thymine, uracil, 2,6-diaminopurine, and hypoxanthine. Examples of nucleobase analogs are 5-azacytosine, 2-chloro-adenine, 5-iodo-cytosine, 8-azaguanine, 5-iodo-uracil, 5-bromo-uracil, 5-fluoro-uracil, 5-ethyl-uracil and 5-trifluoromethyl-uracil.

The term "2'-deoxynucleoside precursors" as used herein, relates to compounds which can be easily converted into 2'-deoxynucleosides by applying methods known in the prior art. Preferred 2'-deoxynucleoside precursors are 2-deoxy-D-ribose (DRI) or carbohydrate compounds which can be converted into the 2-deoxy-D-ribosyl moiety of 2'-deoxynucleosides, for instance, those established in the prior art 1-phospho-2-deoxy-D-ribose, 5-phospho-2-deoxy-D-ribose and those established by the present invention 2-deoxy-D-ribitol, 2-deoxy-D-ribonic acid, 2-deoxy-D-ribono-1,4-lactone, 1-N-morpholino-3,4,5-trihydroxy-pentene-1, and their derivatives.

The method of the invention encompasses methods wherein the decarboxylation step is directly carried out on KDG or its salts or on compounds derived from KDG. Preferred KDG derivatives are 3-deoxy-D-gluconic acid, 3-deoxy-D-mannonic acid, 3-deoxy-D-glucosaminic acid and 3-deoxy-D-mannosaminic acid and their respective salts.

Furthermore, KDG and its salts or protected forms of these wherein one or more of the hydroxyl groups at the positions 4,5 and /or 6 are replaced by protecting groups known for that purpose in the art are also suitable starting materials for the decarboxylation reaction of the present invention. Unless noted otherwise, any reference to KDG in the following specification embraces protected forms of KDG, just as reference to KDG derivatives is intended to embrace protected forms of these derivatives. Similarly, any reference to the products obtained in the methods of the invention is intended to encompass protected forms of these products. Preferred protection groups for the purpose of the invention are those which replace the respective hydroxyl groups by acetate ester, benzoate ester, allyl ether, benzyl ether, trityl ether, ter-butyldimethylsilyl (TBDMS) ether, isopropylidene or a benzylidene acetal.

It should be understood that, depending on suitable reaction conditions for the embodiments of the invention, the carboxylic groups contained in the organic acids used as reactants or obtained as products can be in a protonated form or in their salt form, or may be present in equilibrium. Exemplary salts of these acids are those which have metal or ammonium ions as counterions, particularly alkali metal ions such as sodium and/or potassium.

Most of the carbohydrate compounds and their derivatives described in the present invention exist under several cyclic form but for simplicity reasons have been represented by open chain formulas. It is understood that the present invention encompasses all these isomeric or tautomeric forms.

In a first embodiment of the invention, KDG or its salts is reacted with hydrogen peroxide and undergoes (oxidative) decarboxylation to 2-deoxy-D-ribonic acid (DRN), a compound of formula (II) or its salts.

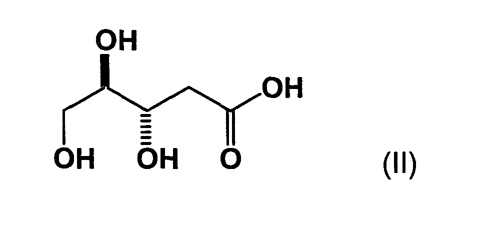

The product may be further converted into or 2-deoxy-D-ribitol (DRL), represented by formula (IV) 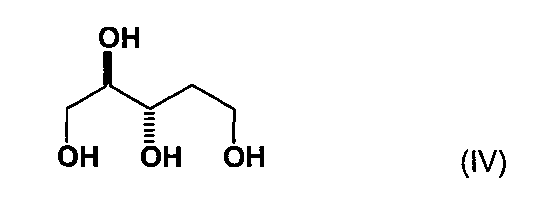 or 2-deoxy-D-ribose (DRI), represented by formula (III) 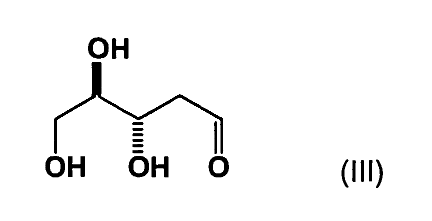 DRN, DRL and particularly DRI are among preferred 2'-deoxynucleoside precursors for the purpose of the present invention. Conversion of DRN to DRI may proceed directly or via DRL as an intermediate.

Preferably, the preparation of DRN is carried out by oxidative decarboxylation of sodium or potassium 2-dehydro-3-deoxy-D-gluconate in aqueous solution with hydrogen peroxide at room temperature as described in example 5. A general method for the preparation of aldonic acids by oxidative decarboxylation of 2-ketoaldonic acids is described in patent EP 1 038 860 A1.

Preferably, the preparation of DRL is carried out by hydrogenation of 2-deoxy-D-ribonolactone in aqueous solution with Rhodium catalyst on carbon at a temperature of 130°C under a pressure of 80 bars as described in example 6. 2-Deoxy-D-ribonolactone can be easily prepared by converting a 2-deoxy-D-ribonate (DRN salt) into 2-deoxy-D-ribonic acid, which is in equilibrium with its lactonic form in aqueous solutions (Han, Tetrahedron. 1993. 49, 349-362; Han, Tetrahedron Asymmetry. 1994. 5, 2535-62).

Preferably the preparation of 2-deoxy-D-ribose (DRI) is carried out by oxidization of 2-deoxy-D-ribitol (DLR), e.g. with chromium oxide in pyridine.

In another embodiment of the invention, decarboxylation takes place by reacting (KDG) or its salts with an amino group-containing reagent Y-H leading to a compound of formula (V). 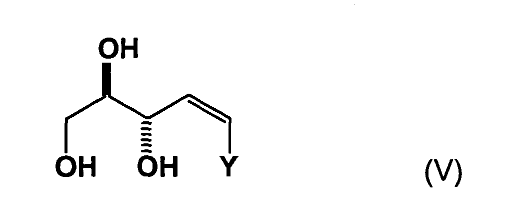 or its respective trans isomer or a protected form thereof, as a 2'-deoxynucleoside precursor. Y-H represents an amine with the hydrogen atom H bound to the nitrogen of the amino group.

In a preferred embodiment of the invention, the amino group-containing reagent represented by Y-H is a linear or cyclic secondary amine; a primary amine that possess a β-carbonyl group, preferably 3-amino-2-indolinone which was found to be effective for the decarboxylation of α-keto acids (Hanson, J. Chem. Education, 1987, 591-595). In each of these cases, -Y in formula (V) represents the respective nitrogen containing residue derived from these amino-group containing reagent.

Preferably, the compound of formula (V) represents an enamine produced via reaction of a linear or cyclic secondary amine as Y-H.

Preferred cyclic secondary amines are morpholine, pyrrolidine, piperidine, or N-methyl piperazine; preferred non-cyclic amines are those of the formula R₁-NH-R₂, wherein R₁ and R₂ independently represent a linear or branched alkyl group of 1-8, preferably 1 to 4 carbon atoms. Particularly preferred as a non-cyclic amine is diethylamine.
Particularly preferred as a cyclic amine is morpholine.

The compound of formula (V) or its trans isomer or a protected form thereof can be further reacted with Z-H, wherein H represents a hydrogen atom and Z represents a leaving group, to produce a compound of formula (VI) 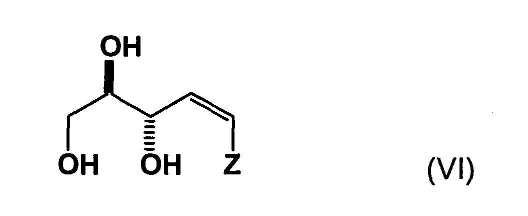 or its respective trans isomer or a protected form thereof, as a 2'-deoxynucleoside precursor. Z-H is preferably water, in which case the compound of formula (VI) is DRI or a protected form thereof (keto-enol-tautomerism).

Preferably, the preparation of the compound of formula (V) is carried out by reacting KDG in benzene with the amine, e.g. morpholine under reflux using the method described in example 7, leading to 1-N-morpholino-3,4,5-trihydroxy-pentene-1. Acid catalysed hydrolysis with water yields 2-deoxy-D-ribose (DRI)

A general route to aldehydes via enamines from α―oxocarboxylic acids carrying β-hydrogens is described by Stamos (Tetrahedron Lett. 23 (1982), 459-462). Other methods for the preparation and hydrolysis of enamines have been described elsewhere (Stork, J. Am. Chem. Soc. 85 (1963), 207-222; Stamhuis, J. Org. Chem. 30 (1965), 2156-2160).

In another embodiment of the invention, KDG or its salt is converted to 3-deoxy-D-gluconic acid (DGN) and/or 3-deoxy-D-mannonic acid (DMN) represented by formula (VII) or the salts of these compounds

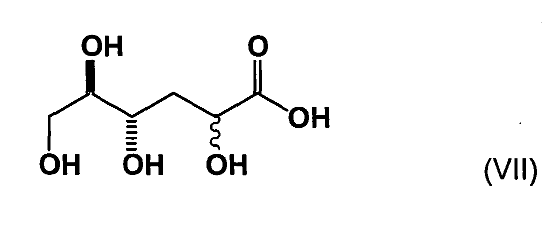

The products resulting from this reaction undergo (oxidative) decarboxylation, preferably using hydrogen peroxide, to yield DRI. Production of a mixture of DGN and DMN or their salts takes place from KDG or its salts by reduction.

Preferably the preparation of 2-deoxy-D-ribose (DRI) is carried out by non-stereoselective reduction of 2-dehydro-3-deoxy-D-gluconic acid in water with sodium borohydride at room temperature using the method described for 2-keto-3-deoxyheptonic acid by Weissbach (J. Biol. Chem. 234 (1959), 705-709), followed by oxidative decarboxylation of 3-deoxy-D-gluconate and 3-deoxy-D-mannonate with hydrogen peroxide as described e.g. in US patent 3,312,683; Richards J. Chem. Soc. (1954), 3638-3640; Sowden J. Am. Chem. Soc. 76 (1954), 3541-3542.

In another embodiment of the invention, KDG or its salt is converted to 3-deoxy-D-glucosaminate (DGM) or 3-deoxy-D-mannosaminate (DMM) represented by formula (VIII) or the salts of these compounds 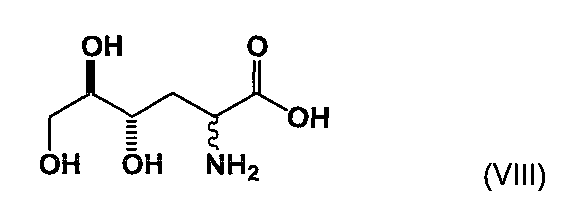 The products resulting from this reaction undergo (oxidative) decarboxylation, preferably using ninhydrin, to yield DRI. Production of a mixture of DGM and DMM or their salts takes place from KDG or its salts by reductive amination.

Preferably the preparation of 2-deoxy-D-ribose is carried out by non-stereoselective reductive amination of sodium or potassium 2-dehydro-3-deoxy-D-gluconate in aqueous solution with ammonia and sodium cyanoborohydride at room temperature, followed by oxidative decarboxylation of 3-deoxy-D-2-glucosaminate and 3-deoxy-D-2-mannosaminate with ninhydrin using the method described for the synthesis of 2-deoxy-D-allose by Shelton (J. Am. Chem. Soc. 118 (1996), 2117-2125; and Borch, J. Am. Chem. Soc. 93 (1971), 2897; Durrwachter, J. Am. Chem. Soc. 108 (1986), 7812 referenced therein).

Another aspect of the invention is a convenient and cost-effective method for preparing KDG either from D-gluconate (GCN) or from D-glucosaminate through the use of recombinant enzymes.

In a preferred embodiment of the method of the invention, the compound of formula (I) is produced in a preliminary step from a D-gluconate salt by the use of a D-gluconate dehydratase activity. Preferred salts are potassium or sodium D-gluconate. Preferably the D-gluconate dehydratase is encoded by a polynucleotide comprising the nucleotide sequence selected from the group consisting of:
(a) nucleotide sequences encoding a polypeptide comprising the amino acid sequence of SEQ ID N°2;
(b) nucleotide sequences comprising the coding sequence of SEQ ID N°1;
(c) nucleotide sequences encoding a fragment encoded by a nucleotide sequence of (a) or (b);
(d) nucleotide sequences hybridising with a nucleotide sequence of any one of (a) to (c); and
(e) nucleotide sequences which deviate from the nucleoside sequence of (d) as a result of degeneracy of the genetic code.

The enzymatic synthesis of KDG or its salts using D-gluconate dehydratase proceeds according to the following reaction: D-gluconate is converted into KDG by the elimination of one water molecule. The activity of a D-gluconate dehydratase has been characterized in different bacteria species e.g. in *Alcaligenes* (Kersters, Methods in Enzymology 42 (1975), 301-304); *Clostridium pasteurianum,* (Gottschalk, Methods in Enzymology 90 (1982), 283-287); *Thermoplasma acidophilum* (Budgen, FEBS Letters 196 (1986), 207-210) and *Sulfolobus solfataricus* (Nicolaus, Biotechnology Letters 8(7) (1986), 497-500). The preferred D-gluconate dehydratase was identified by screening several collection strains for D-gluconate dehydratase activity. The gene encoding a D-gluconate dehydratase, which was designated gcnD was selected from a genomic library of *Agrobacterium tumefaciens* strain C58, and further inserted in a multi copy vector optimised for expression. It was shown that a crude extract from E. coli cells over-expressing the gcnD gene catalysed the total conversion of D-gluconate into KDG (see Example 2).

In a further preferred embodiment of the method of the invention, the compound of formula (I) is produced in a preliminary step from D-glucosaminate by the use of a D-glucosaminate deaminase activity. Preferably the D-glucosaminate deaminase is encoded by a polynucleotide comprising the nucleotide sequence selected from the group consisting of:
(f) nucleotide sequences encoding a polypeptide comprising the amino acid sequence of SEQ ID N°4;
(g) nucleotide sequences comprising the coding sequence of SEQ ID N°3;
(h) nucleotide sequences encoding a fragment encoded by a nucleotide sequence of (a) or (b);
(i) nucleotide sequences hybridising with a nucleotide sequence of any one of (a) to (c); and
(j) nucleotide sequences which deviate from the nucleoside sequence of (d) as a result of degeneracy of the genetic code.

The enzymatic synthesis of KDG or its salts using D-glucosaminate deaminase proceeds according to the following reaction: D-glucosaminate is converted into KDG by the elimination of one molecule water and one molecule of ammonia. The activity of a D-glucosaminate deaminase has been characterized in different bacteria species e.g. in *Pseudomonas fluorescens* (Iwamoto, Agric. Biol. Chem. 53 (1989), 2563-2569) *Agrobacterium radiobacter* (Iwamoto, FEBS Letters 104 (1979), 131-134; Iwamoto, J. Biochem. 91 (1982), 283-289), and its requirement for Mn²⁺ ion was shown (Iwamoto, Biosci. Biotech. Biochem. 59 (1995), 408-411).

The preferred D-glucosaminate deaminase was identified by screening several collection strains for D-glucosaminate deaminase activity. The gene encoding a D-glucosaminate deaminase, which was designated gmaA was isolated from *Agrobacterium tumefaciens* strain C58 by cloning a gene annotated as a putative D-serine deaminase. The gmaA gene was further inserted in a multi copy vector optimised for expression. It was shown that a crude extract from E. coli cells over-expressing the gmaA gene catalysed the conversion of D-glucosaminate into KDG (see Example 4).

These and other embodiments are disclosed and encompassed by the description and examples of the present invention. Further literature concerning any one of the methods, uses and compounds to be employed in accordance with the present invention may be retrieved from public libraries, using for example electronic devices.

For example the public database "Medline" may be utilized which is available on the Internet, for example under http://www.ncbi.nlm.nih.gov/PubMed/medline.html. Further databases and addresses, such as http://www.ncbi.nlm.nih.gov/, http://www.infobiogen.fr/, http://www.fmi.ch/biology/research_tools.html, http://www.tigr.org/, are known to the person skilled in the art and can also be obtained using, e.g., http://www.google.de. An overview of patent information in biotechnology and a survey of relevant sources of patent information useful for retrospective searching and for current awareness is given in Berks, TIBTECH 12 (1994), 352-364.
Furthermore, the term "and/or" when occurring herein includes the meaning of "and", "or" and "all or any other combination of the elements connected by said term".

### EXAMPLES

### Example 1. Cloning of a gene encoding a D-gluconate dehydratase from Agrobacterium tumefaciens strain C58 (CIP 104333)

*Agrobacterium tumefaciens* strain C58 (CIP 104333) was obtained from Institut Pasteur Collection (CIP, Paris, France). Chromosomal DNA was extracted and a D-gluconate dehydratase gene was amplified by PCR according to standard protocols using the following primers:
5'-CCCTTAATTAATGACGACATCTGATAATCTTC-3', depicted in SEQ ID N° 5;
5'-TTTGCGGCCGCTTAGTGGTTATCGCGCGGC-3', depicted in SEQ ID N° 6;
5'-CCCGGTACCATGACGACATCTGATAATCTTC-3',depicted in SEQ ID N° 7;
A first DNA fragment amplified using the two primers depicted in SEQ ID N° 5 and SEQ ID N° 6, was ligated into a pUC18-derived vector previously digested by PacI and Notl to yield the plasmid pVDM80. A second DNA fragment amplified using the two primers depicted in SEQ ID N° 6 and SEQ ID N° 7, was ligated into a pET29a vector (Novagen) previously digested by KpnI and NotI to yield the plasmid pVDM82. The nucleotide sequence of the cloned gene is depicted in SEQ ID N° 1 and the sequence of the polypeptide encoded by this gene is depicted in SEQ ID N° 2.

### Example 2. Expression of a D-gluconate dehydratase activity in Escherichia coli and preparation of 2-dehydro-3-deoxy-D-gluconate from D-gluconate.

Competent cells of E. coli BL21 were transformed with the pVDM82 plasmid constructed as described in example 1 yielding strain +1289. Strain + 1289 was cultivated at 30°C in Luria-Bertani (LB) medium (Difco) containing 30 mg/l kanamycin until OD(600 nm) reached a value of 0.6. Then isopropyl-β-D-thiogalactopyranoside (IPTG) was added to a 0.5 mM final concentration. After a further cultivation period of 2 hours and 30 minutes, cells were collected by centrifugation and washed once with 20 mM sodium phosphate buffer pH 7.2. A cell extract was prepared by suspending about 5 g of cells in 10 ml of Tris-HCl 50 mM pH 8.5 buffer containing 10000 units lysozyme (Ready-Lyse, Epicentre, Madison, Wisconsin) and 1 mM EDTA, and incubating the suspension at 30°C for 15 minutes. Then 10000 kUnits deoxyribonuclease I (DNase I, Sigma) as well as 5 mM MgCl2 were added to the preparation which was incubated at 30°C for an additional period of 15 minutes. The cell extract thus obtained was kept frozen at -20°C before use.
1.5 ml of the cell extract was mixed with 2M sodium or potassium D-gluconate in a total volume of 10 ml. This preparation was incubated at 37°C after the pH has been adjusted to 8.5. The progression of 2-dehydro-3-deoxy-D-gluconate (KDG) synthesis was followed by analysing aliquots taken after increasing periods of incubation. Several dilution parts of these aliquots were deposited on silica plates and chromatographied in the following solvent system: isopropanol / water (90/10). A yellow spot of KDG (Rf ∼0.40) was detected after revelation with p-anisaldehyde. KDG was also quantitated using a spectrophotometric assay based on the reaction with semicarbazide hydrochloride as described by Mac Gee (J. Biol. Chem. 1954. 210, 617-626). Typically, after a 30h period of incubation and using the spectrophotometric assay, KDG concentration ranged from 1.5 to 2 M.
The sodium or potassium 2-dehydro-3-deoxy-D-gluconate solution thus obtained could be used as such for further synthetic steps. 2-Dehydro-3-deoxy-D-gluconic acid could also be prepared from such a solution applying published protocols (Bender, Anal. Biochem. 1974. 61, 275-279). A crude preparation of a mixture of 2-dehydro-3-deoxy-D-gluconic acid and KCI could also be obtained by adding one equivalent of HCl to a potassium 2-dehydro-3-deoxy-D-gluconate solution which was then evaporated.

### Example 3. Cloning of a gene encoding a D-glucosaminate deaminase from Agrobacterium tumefaciens strain C58 (CIP 104333)

*Agrobacterium tumefaciens* strain C58 (CIP 104333) was obtained from Institut Pasteur Collection (CIP, Paris, France). Chromosomal DNA was extracted and a D-glucosaminate deaminase gene was amplified by PCR according to standard protocols using the following primers:
5'-CCCTTAATTAATGCAGTCTTCTTCAGCTCTTC-3', depicted in SEQ ID N° 8;
5'-TTTGCGGCCGCCTAGTGAAAGAAGGTTGTGTAGAT-3', depicted in SEQ ID N° 9;
5'-AAATCATGACTATGCAGTCTTCTTCAGCTCTTCG-3', depicted in SEQ ID N° 10;
5'-TATAGATCTCTAGTGAAAGAAGGTTGTGTAGAT-3', depicted in SEQ ID N° 11;
A first DNA fragment amplified using the two primers depicted in SEQ ID N° 8 and SEQ ID N° 9, was ligated into a pUC18-derived vector previously digested by Pacl and Notl to yield the plasmid pKDGb1. A second DNA fragment amplified using the two primers depicted in SEQ ID N° 10 and SEQ ID N° 11, was ligated into a pQE60 vector (Qiagen) previously digested by BspH1 and BglII to yield the plasmid pEP18. The nucleotide sequence of the cloned gene is depicted in SEQ ID N° 3 and the sequence of the polypeptide encoded by this gene is depicted in SEQ ID N° 4.

### Example 4. Expression of a D-glucosaminate deaminase activity in Escherichia coli and preparation of 2-dehydro-3-deoxy-D-gluconic acid from D-glucosaminate

Competent cells of E. coli MG1655 were transformed with the pEP18 plasmid constructed as described in example 1 and pREP4 (Qiagen) yielding strain +1068. Strain + 1068 was cultivated at 37°C in LB medium containing 30 mg/l kanamycin and 100 mg/l ampicillin until OD(600 nm) reached a value of 0.6. Then IPTG was added to a 0.5 mM final concentration. After a further cultivation period of 2 hours and 30 minutes, cells were collected by centrifugation and washed once with 20 mM sodium phosphate buffer pH 7.2. A cell extract was prepared using the protocol described in example 2.

2 ml of the cell extract was mixed with 100 mM sodium or potassium D-glucosaminate and 0.1 mM pyridoxal phosphate in a total volume of 5 ml. This preparation was incubated at 37°C after the pH has been adjusted to 7.5.
The progression of 2-dehydro-3-deoxy-D-gluconate (KDG) synthesis was followed using the protocols described in example 2. Typically, after a 30h period of incubation and using the spectrophotometric assay described in example 2, KDG concentration ranged from 50 to 100 mM.

### Example 5. Preparation of 2-deoxy-D-ribonate from 2-dehydro-3-deoxy-D-gluconate

0.5 ml of a 31% hydrogen peroxyde solution were added to 5 ml of a 1M potassium 2-dehydro-3-deoxy-D-gluconate (KDG) solution at 25°C. The progression of KDG decarboxylation was followed both by the observation of bubbles resulting from the release of carbon dioxide and by the disappearance of KDG using the thin layer chromatography protocol described in example 2. Typically, after a 3h period of reaction the concentration of residual KDG was less than 10 mM.

### Example 6. Preparation of 2-deoxy-D-ribitol from 2-deoxy-D-ribonolactone

0.2 g of Rhodium ( 5 % on carbon) catalyst was added to an aqueous solution of 1 g 2-deoxy-D-ribonolactone prepared following a method described by Deriaz (J. Chem. Soc. (1949), 1879-1883) for the synthesis of 2-deoxy-L-ribonolactone. Hydrogenation of 2-deoxy-D-ribonolactone was performed at 130°C under a pressure of 80 bars. The solution obtained after filtration of the reaction mixture was evaporated. The residue was dissolved in ethyl acetate and further purified by chromatography on a silica column. The solvent was removed in vacuo leading to a yellow oil (yield 85%).The compound thus obtained was identical with 2-deoxy-D-ribitol obtained by reduction of 2-deoxy-D-ribose as described by Rabow (J. Am. Chem. Soc. 122 (1999), 3196-3203).

### Example 7. Preparation of 1-N-morpholino-3,4,5-trihydroxypentene-1 from 2-dehydro-3-deoxy-D-gluconate.

2 g of 2-dehydro-3-deoxy-D-gluconic acid were suspended in 150 ml benzene. 1.1 ml morpholine and 100 mg p-toluenesulfonic acid were added to the suspension and the reaction mixture was refluxed for 3 hours. Water formed by this reaction was removed by distillation. Benzene was decanted. Solid compounds attached to the vessel were collected, washed with acetone and dried. The main compound present in this preparation (yield 40%) was further purified by column chromatography on a silica column using a gradient of methanol in chloroform. Fractions containing 1-N-morpholino-3,4,5-trihydroxypentene-1 were pooled and solvent was removed in vacuo.
¹H-NMR (D₂0): δ = 3.15 ppm (4H, t, morpholine), 3.8 ppm (4H, t, morpholine), 3.4 to 4 ppm, (4H, m, 5a-H, 5b-H, 4-H, 3-H), 6.3 and 6.8 ppm (2H, 2d, 1-H and 2-H, *J* = 4 Hz).

## Claims

1. A method for producing 2'-deoxynucleosides or 2'-deoxynucleoside precursors from a compound of formula (I) or its salts 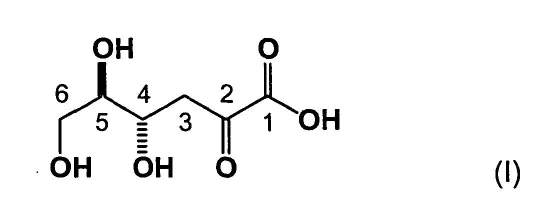 or a protected form thereof in a process comprising a decarboxylation step.

2. The method of claim 1 wherein the decarboxylation step cleaves the C1-C2 bond of the compound of formula (I) or its salts or a protected form thereof.

3. The method of claim 1 or 2, wherein the decarboxylation step is directly carried out on the compound of formula (I) or its salts or a protected form thereof.

4. The method of any of claims 1 to 3, wherein the decarboxylation step takes place by reacting the compound of formula (I) or its salts or a protected form thereof with hydrogen peroxide to yield a compound of formula (II) or its salts 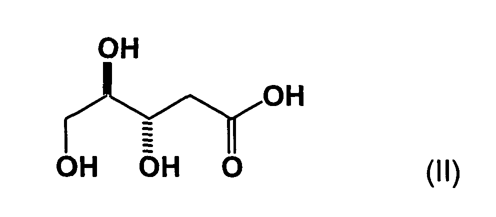 or a protected form thereof as a 2'-deoxynucleoside precursor.

5. The method of claim 4, further comprising the conversion of the compound of formula (II) or its salts or a protected form thereof into a compound of formula (IV) 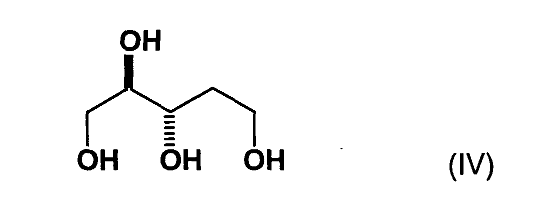 or a protected form thereof as a 2'-deoxynucleoside precursor.

6. The method of claim 4, further comprising the conversion of the compound of formula (II) or its salts or a protected form thereof into a compound of formula (III) 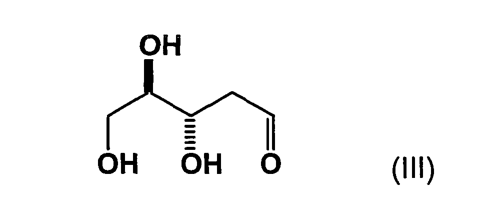 or a protected form thereof as a 2'-deoxynucleoside precursor.

7. The method of claim 6, comprising the conversion of the compound of formula (II) or its salts or a protected form thereof into the compound of formula (IV) or a protected form thereof as an intermediate which is then converted to the compound of formula (III) or a protected form thereof.

8. The method of any of claims 1 to 3, wherein the decarboxylation step takes place by reacting the compound of formula (I) or its salts or a protected form thereof with an amine Y-H, wherein H represents a hydrogen atom bound to the nitrogen atom of the amino group, to produce a compound of formula (V), 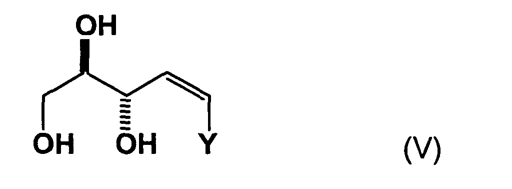 or its respective trans isomer or a protected form thereof, as a 2'-deoxynucleoside precursor.

9. The method of claim 8, wherein Y-H represents a linear or cyclic secondary amine.

10. The method of claims 8 or 9, wherein Y-H is morpholine, pyrrolidine, piperidine, N-methyl piperazine or diethylamine.

11. The method of any of claims 8 to 10, further comprising the step of reacting a compound of formula (V) or its trans isomer or a protected form thereof with Z-H, wherein H represents a hydrogen atom and Z represents a leaving group, to produce a compound of formula (VI) 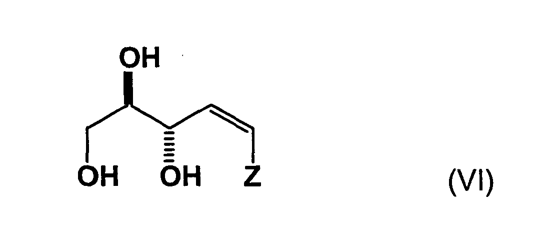 or its respective trans isomer or a protected form thereof, as a 2'-deoxynucleoside precursor.

12. The method of claim 11, wherein Z-H is water, to produce a compound of formula (III) or a protected form thereof as a 2'-deoxynucleoside precursor.

13. The method of claim 1 or 2, wherein the compound of formula (I) or its salts or a protected form thereof is converted to a compound of formula (VII), or its salts or a protected form thereof or a mixture of the respective epimers, 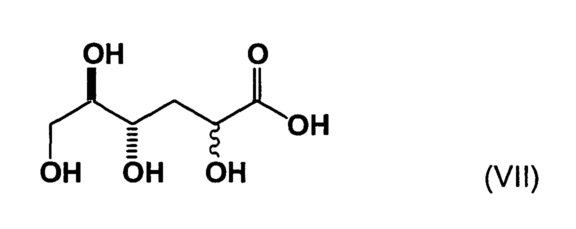 which is then decarboxylated to yield a compound of formula (III) or a protected form thereof as a 2'-deoxynucleoside precursor.

14. The method of claim 13, wherein the conversion of (I) or its salts or a protected form thereof to (VII) or a protected form thereof takes place by reduction with sodium borohydride.

15. The method of claim 13 to 14, wherein the decarboxylation step takes place by reaction with hydrogen peroxide.

16. The method of claim 1 or 2, wherein the compound of formula (I) or its salts or a protected form thereof is converted to a compound of formula (VIII), or its salts or a protected form thereof or a mixture of the respective epimers, 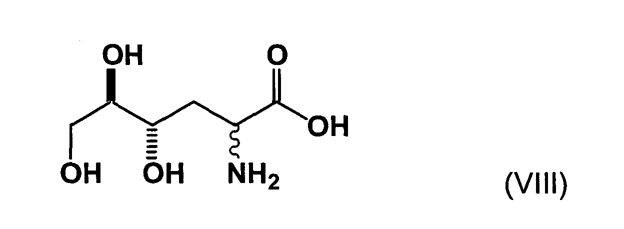 which is then decarboxylated to yield a compound of formula (III) or a protected form thereof as a 2'-deoxynucleoside precursor.

17. The method of claim 16, wherein a compound of formula (VIII) or a protected form thereof or a mixture of the respective epimers is reacted with ninhydrin, thereby leading to the compound (III) or a protected form thereof.

18. The method of claim 16 or 17, wherein the conversion of (I) or its salts or a protected form thereof to (VIII) or a protected form thereof takes place by reductive amination with ammonia and sodium cyanoborohydride.

19. The method of any of claims 1 to 18, wherein the protective group(s) are independently chosen from acetate ester, benzoate ester, allyl ether, benzyl ether, trityl ether, ter-butyldimethylsilyl (TBDMS) ether, isopropylidene or a benzylidene acetal.

20. The method of any one of claims 1 to 19, comprising the preliminary step of producing the compound of formula (I) from a D-gluconate salt by the use of a gluconate dehydratase activity.

21. The method of claim 20, wherein the D-gluconate salt is potassium or sodium D-gluconate.

22. The method of claims 20 or 21, wherein the gluconate dehydratase is encoded by a polynucleotide comprising the nucleotide sequence selected from the group consisting of:
(a) nucleotide sequences encoding a polypeptide comprising the amino acid sequence of SEQ ID N°2;
(b) nucleotide sequences comprising the coding sequence of SEQ ID N°1;
(c) nucleotide sequences encoding a fragment encoded by a nucleotide sequence of (a) or (b);
(d) nucleotide sequences hybridising with a nucleotide sequence of any one of (a) to (c); and
(e) nucleotide sequences which deviate from the nucleoside sequence of (d) as a result of degeneracy of the genetic code.

23. The method of any one of claims 1 to 19, comprising the preliminary step of producing the compound of formula (I) from D-glucosaminate by the use of a glucosaminate deaminase activity.

24. The method of claim 23, wherein the glucosaminate deaminase is encoded by a polynucleotide comprising the nucleotide sequence selected from the group consisting of:
(a) nucleotide sequences encoding a polypeptide comprising the amino acid sequence of SEQ ID N°4;
(b) nucleotide sequences comprising the coding sequence of SEQ ID N°3;
(c) nucleotide sequences encoding a fragment encoded by a nucleotide sequence of (a) or (b);
(d) nucleotide sequences hybridising with a nucleotide sequence of any one of (a) to (c); and
(e) nucleotide sequences which deviate from the nucleoside sequence of (d) as a result of degeneracy of the genetic code.

25. Use of a polynucleotide as defined in claim 22 or of a gluconate dehydratase encoded by such a polynucleotide in a method according to claims 20 or 21.

26. Use of a polynucleotide as defined in claim 24 or of a glucosaminate deaminase encoded by such a polynucleotide in a method according to claim 23.


SEQUENCE LISTING

<110>  Evologic S.A., MARLIERE Philippe

<120>  PRODUCTION OF 2'-DEOXYNUCLEOSIDES AND 2'-DEOXYNUCLEOSIDE PRECURSORS
FROM 2-DEHYDRO-3-DEOXY-D-GLUCONATE

<130>  G 3111 EP

<160>  11

<170>  PatentIn version 3.1

<210>  1
<211>  1812
<212>  DNA
<213>  Agrobacterium tumefaciens

<220>
<221>  CDS
<222>  (1)..(1809)
<223>

<400>  1
atg acg aca tct gat aat ctt cct gca act cag ggc aag ctc cgt tcg      48
Met Thr Thr Ser Asp Asn Leu Pro Ala Thr Gln Gly Lys Leu Arg Ser
1               5                   10                  15

cgc gcc tgg ttc gac aac cca gcc aat gcg gac atg acc gcg ctt tat      96
Arg Ala Trp Phe Asp Asn Pro Ala Asn Ala Asp Met Thr Ala Leu Tyr
            20                  25                  30

ctc gag cgt tac atg aac ttc ggt ctc agc cag gcc gag ctt cag tcc      144
Leu Glu Arg Tyr Met Asn Phe Gly Leu Ser Gln Ala Glu Leu Gln Ser
        35                  40                  45

gac cgc ccg att atc ggt att gcg cag acc ggt tcc gac ctt tcg ccc      192
Asp Arg Pro Ile Ile Gly Ile Ala Gln Thr Gly Ser Asp Leu Ser Pro
    50                  55                  60

tgc aac cgt cat cat ctg gaa ctc gcc aac cgt ctg cgt gaa ggc att      240
Cys Asn Arg His His Leu Glu Leu Ala Asn Arg Leu Arg Glu Gly Ile
65                  70                  75                  80

cgt gaa gcc ggc ggc atc gcc atc gaa ttc ccg gtg cat ccg atc cag      288
Arg Glu Ala Gly Gly Ile Ala Ile Glu Phe Pro Val His Pro Ile Gln
                85                  90                  95

gaa acc ggt aag cgt ccg aca gcg ggc ctt gat cgc aac ctg gct tac 336
Glu Thr Gly Lys Arg Pro Thr Ala Gly Leu Asp Arg Asn Leu Ala Tyr
            100                 105                 110

ctc ggc ctc gtg gaa gtg ctt tat ggc tat ccg ctc gac ggc gtt gtt 384
Leu Gly Leu Val Glu Val Leu Tyr Gly Tyr Pro Leu Asp Gly Val Val
        115                 120                 125

ctg acc atc ggc tgc gac aag acc acg cct gcc tgt ctt atg gcg gcg 432
Leu Thr Ile Gly Cys Asp Lys Thr Thr Pro Ala Cys Leu Met Ala Ala
    130                 135                 140

gcc acc gtc aac att ccg gcc atc gcc ctt tcc gtc ggt ccc atg ctg 480
Ala Thr Val Asn Ile Pro Ala Ile Ala Leu Ser Val Gly Pro Met Leu
145                 150                 155                 160

aac ggc tgg ttc cgc ggt gag cgc acc ggt tcc ggc acc atc gtc tgg 528
Asn Gly Trp Phe Arg Gly Glu Arg Thr Gly Ser Gly Thr Ile Val Trp
                165                 170                 175

aag gcc cgc gaa ctg ctg gcg aag ggc gag atc gat tac cag ggc ttc 576
Lys Ala Arg Glu Leu Leu Ala Lys Gly Glu Ile Asp Tyr Gln Gly Phe
            180                 185                 190

gtc aag ctc gtt gcc tcg tct gcc ccg tcc acc ggc tat tgc aac acc 624
Val Lys Leu Val Ala Ser Ser Ala Pro Ser Thr Gly Tyr Cys Asn Thr
        195                 200                 205

atg ggc acg gca aca acc atg aac tcg ctc gcc gaa gcg ctc ggc atg 672
Met Gly Thr Ala Thr Thr Met Asn Ser Leu Ala Glu Ala Leu Gly Met
    210                 215                 220

cag ctt ccc ggc tcc gcc gcc att ccg gcg cct tac cgt gac cgt cag 720
Gln Leu Pro Gly Ser Ala Ala Ile Pro Ala Pro Tyr Arg Asp Arg Gln
225                 230                 235                 240

gaa gtc tct tac ctc acc ggc ctg cgc atc gtc gac atg gtc agg gaa 768
Glu Val Ser Tyr Leu Thr Gly Leu Arg Ile Val Asp Met Val Arg Glu
                245                 250                 255

gac ctg aaa cca tca gac atc atg acc aag gat gcc ttc atc aac gcc 816
Asp Leu Lys Pro Ser Asp Ile Met Thr Lys Asp Ala Phe Ile Asn Ala
            260                 265                 270

atc cgc gtt aat tcg gcg atc ggc ggt tcc acc aac gcg ccg atc cat 864
Ile Arg Val Asn Ser Ala Ile Gly Gly Ser Thr Asn Ala Pro Ile His
        275                 280                 285

cta aac ggc ctt gcc cgc cat gtc ggc gtc gag ctg acg gtg gat gac 912
Leu Asn Gly Leu Ala Arg His Val Gly Val Glu Leu Thr Val Asp Asp
    290                 295                 300

tgg cag acc tat ggc gaa gac gtg ccg ctg ctc gtc aac ctg cag ccg 960
Trp Gln Thr Tyr Gly Glu Asp Val Pro Leu Leu Val Asn Leu Gln Pro
305                 310                 315                 320

gca ggc gaa tat ctc ggc gag gac tat tac cat gcc ggc ggc gtt ccc 1008
Ala Gly Glu Tyr Leu Gly Glu Asp Tyr Tyr His Ala Gly Gly Val Pro
                325                 330                 335

gct gtc gtc aat cag ctg atg acc caa ggg ctg atc atg gaa gac gcc 1056
Ala Val Val Asn Gln Leu Met Thr Gln Gly Leu Ile Met Glu Asp Ala
            340                 345                 350

atg acc gtc aac ggc aag acc atc ggc gac aat tgc cgt ggc gcg atc 1104
Met Thr Val Asn Gly Lys Thr Ile Gly Asp Asn Cys Arg Gly Ala Ile
        355                 360                 365

atc gaa gac gag aag gtc atc cgc ccc tat gag cag ccg ctc aag gag 1152
Ile Glu Asp Glu Lys Val Ile Arg Pro Tyr Glu Gln Pro Leu Lys Glu

370                    375          .          380

cgt gcc ggc ttc cgc gtt ctg cgc ggc aat ctg ttc tcc tcg gcc atc        1200
Arg Ala Gly Phe Arg Val Leu Arg Gly Asn Leu Phe Ser Ser Ala Ile
385                 390                 395                 400

atg aag aca agc gtg att tcg gaa gaa ttc cgc ggt cgt tac ctc tcc        1248
Met Lys Thr Ser Val Ile Ser Glu Glu Phe Arg Gly Arg Tyr Leu Ser
                405                 410                 415

aac cct gat gat ccg gaa gcc ttc gaa ggc cgc gcc gtg gtg ttc gat        1296
Asn Pro Asp Asp Pro Glu Ala Phe Glu Gly Arg Ala Val Val Phe Asp
            420                 425                 430

ggt ccg gag gat tac cat cat cgc atc gac gat ccg tcg ctt ggc atc        1344
Gly Pro Glu Asp Tyr His His Arg Ile Asp Asp Pro Ser Leu Gly Ile
        435                 440                 445

gac gcc aac acc gtc ctg ttc atg cgc ggc gcc ggt ccg atc ggt tac        1392
Asp Ala Asn Thr Val Leu Phe Met Arg Gly Ala Gly Pro Ile Gly Tyr
    450                 455                 460

ccg ggc gca gcg gaa gtg gtg aac atg cgc gcg ccg gat tac ctt ctg        1440
Pro Gly Ala Ala Glu Val Val Asn Met Arg Ala Pro Asp Tyr Leu Leu
465                 470                 475                 480

aag caa ggc gtc agt tcg ctg ccc tgc atc ggc gat ggc cgc cag tcc        1488
Lys Gln Gly Val Ser Ser Leu Pro Cys Ile Gly Asp Gly Arg Gln Ser
                485                 490                 495

ggc acg tcg ggc agc cca tcc atc ctc aat gcc tcg ccg gaa gcg gcg        1536
Gly Thr Ser Gly Ser Pro Ser Ile Leu Asn Ala Ser Pro Glu Ala Ala
            500                 505                 510

gcc ggc ggc ggt ctg tct att ctg cag acg ggt gac cgc gtc cgc atc        1584
Ala Gly Gly Gly Leu Ser Ile Leu Gln Thr Gly Asp Arg Val Arg Ile
        515                 520                 525

gat gtg ggc cgc ggc aag gcc gat atc ctg ata tca ggt gaa gag ctc        1632
Asp Val Gly Arg Gly Lys Ala Asp Ile Leu Ile Ser Gly Glu Glu Leu
    530                 535                 540

gcc aag cgt tac gag gcg ctg gca gct cag ggc ggt tat aag ttc ccc        1680
Ala Lys Arg Tyr Glu Ala Leu Ala Ala Gln Gly Gly Tyr Lys Phe Pro
545                 550                 555                 560

gac cac cag acg ccg tgg cag gaa atc cag cgc ggt atc gtc agc cag        1728
Asp His Gln Thr Pro Trp Gln Glu Ile Gln Arg Gly Ile Val Ser Gln
                565                 570                 575

atg gaa acc ggc gcg gtt ctg gaa ccg gcc gta aag tat cag cgc atc        1776
Met Glu Thr Gly Ala Val Leu Glu Pro Ala Val Lys Tyr Gln Arg Ile
            580                 585                 590

gcc cag acc aag ggc ctg ccg cgc gat aac cac tga                        1812
Ala Gln Thr Lys Gly Leu Pro Arg Asp Asn His
        595                 600

<210>  2

<211>  603

<212>  PRT

<213>  Agrobacterium tumefaciens

<400>  2

Met Thr Thr Ser Asp Asn Leu Pro Ala Thr Gln Gly Lys Leu Arg Ser
1               5                   10                  15

Arg Ala Trp Phe Asp Asn Pro Ala Asn Ala Asp Met Thr Ala Leu Tyr
            20                  25                  30

Leu Glu Arg Tyr Met Asn Phe Gly Leu Ser Gln Ala Glu Leu Gln Ser
        35                  40                  45

Asp Arg Pro Ile Ile Gly Ile Ala Gln Thr Gly Ser Asp Leu Ser Pro
    50                  55                  60

Cys Asn Arg His His Leu Glu Leu Ala Asn Arg Leu Arg Glu Gly Ile
65                  70                  75                  80

Arg Glu Ala Gly Gly Ile Ala Ile Glu Phe Pro Val His Pro Ile Gln
                85                  90                  95

Glu Thr Gly Lys Arg Pro Thr Ala Gly Leu Asp Arg Asn Leu Ala Tyr
            100                 105                 110

Leu Gly Leu Val Glu Val Leu Tyr Gly Tyr Pro Leu Asp Gly Val Val
        115                 120                 125

Leu Thr Ile Gly Cys Asp Lys Thr Thr Pro Ala Cys Leu Met Ala Ala
    130                 135                 140

Ala Thr Val Asn Ile Pro Ala Ile Ala Leu Ser Val Gly Pro Met Leu
145                 150                 155                 160

Asn Gly Trp Phe Arg Gly Glu Arg Thr Gly Ser Gly Thr Ile Val Trp
                165                 170                 175

Lys Ala Arg Glu Leu Leu Ala Lys Gly Glu Ile Asp Tyr Gln Gly Phe
            180                 185                 190

Val Lys Leu Val Ala Ser Ser Ala Pro Ser Thr Gly Tyr Cys Asn Thr
        195                 200                 205

Met Gly Thr Ala Thr Thr Met Asn Ser Leu Ala Glu Ala Leu Gly Met
    210                 215                 220

Gln Leu Pro Gly Ser Ala Ala Ile Pro Ala Pro Tyr Arg Asp Arg Gln
225                 230                 235                 240

Glu Val Ser Tyr Leu Thr Gly Leu Arg Ile Val Asp Met Val Arg Glu
                245                 250                 255

Asp Leu Lys Pro Ser Asp Ile Met Thr Lys Asp Ala Phe Ile Asn Ala
            260                 265                 270

Ile Arg Val Asn Ser Ala Ile Gly Gly Ser Thr Asn Ala Pro Ile His

275                 280                 285

Leu Asn Gly Leu Ala Arg His Val Gly Val Glu Leu Thr Val Asp Asp
    290                 295                 300

Trp Gln Thr Tyr Gly Glu Asp Val Pro Leu Leu Val Asn Leu Gln Pro
305                 310                 315                 320

Ala Gly Glu Tyr Leu Gly Glu Asp Tyr Tyr His Ala Gly Gly Val Pro
                325                 330                 335

Ala Val Val Asn Gln Leu Met Thr Gln Gly Leu Ile Met Glu Asp Ala
            340                 345                 350

Met Thr Val Asn Gly Lys Thr Ile Gly Asp Asn Cys Arg Gly Ala Ile
        355                 360                 365

Ile Glu Asp Glu Lys Val Ile Arg Pro Tyr Glu Gln Pro Leu Lys Glu
    370                 375                 380

Arg Ala Gly Phe Arg Val Leu Arg Gly Asn Leu Phe Ser Ser Ala Ile
385                 390                 395                 400

Met Lys Thr Ser Val Ile Ser Glu Glu Phe Arg Gly Arg Tyr Leu Ser
                405                 410                 415

Asn Pro Asp Asp Pro Glu Ala Phe Glu Gly Arg Ala Val Val Phe Asp
            420                 425                 430

Gly Pro Glu Asp Tyr His His Arg Ile Asp Asp Pro Ser Leu Gly Ile
        435                 440                 445

Asp Ala Asn Thr Val Leu Phe Met Arg Gly Ala Gly Pro Ile Gly Tyr
    450                 455                 460

Pro Gly Ala Ala Glu Val Val Asn Met Arg Ala Pro Asp Tyr Leu Leu
465                 470                 475                 480

Lys Gln Gly Val Ser Ser Leu Pro Cys Ile Gly Asp Gly Arg Gln Ser
                485                 490                 495

Gly Thr Ser Gly Ser Pro Ser Ile Leu Asn Ala Ser Pro Glu Ala Ala
            500                 505                 510

Ala Gly Gly Gly Leu Ser Ile Leu Gln Thr Gly Asp Arg Val Arg Ile
        515                 520                 525

Asp Val Gly Arg Gly Lys Ala Asp Ile Leu Ile Ser Gly Glu Glu Leu
    530                 535                 540

Ala Lys Arg Tyr Glu Ala Leu Ala Ala Gln Gly Gly Tyr Lys Phe Pro
545                 550                 555                 560

Asp His Gln Thr Pro Trp Gln Glu Ile Gln Arg Gly Ile Val Ser Gln
                565                 570                 575

Met Glu Thr Gly Ala Val Leu Glu Pro Ala Val Lys Tyr Gln Arg Ile
            580                 585                 590

Ala Gln Thr Lys Gly Leu Pro Arg Asp Asn His
        595                 600

<210>  3

<211>  1272

<212>  DNA

<213>  Agrobacterium tumefaciens

<220>

<221>  CDS

<222>  (1)..(1269)

<223>

<400>  3
atg cag tct tct tca gct ctt cgg caa tca acc ggc gat cag tcg gaa        48
Met Gln Ser Ser Ser Ala Leu Arg Gln Ser Thr Gly Asp Gln Ser Glu
1               5                   10                  15

tac cat gcc cag tcg aat atg atc ggc tct agc ccg gcg gac ggt ttg        96
Tyr His Ala Gln Ser Asn Met Ile Gly Ser Ser Pro Ala Asp Gly Leu
            20                  25                  30

ctc gca ttg ccg ctt ctg acc gtc gat ctt gcc gtc tat cgc ggt aat       144
Leu Ala Leu Pro Leu Leu Thr Val Asp Leu Ala Val Tyr Arg Gly Asn
        35                  40                  45

cgg gat cgc ttt ctt gcg ctt gtc tcg gcc cat gga gcg aag gcg gct       192
Arg Asp Arg Phe Leu Ala Leu Val Ser Ala His Gly Ala Lys Ala Ala
    50                  55                  60

cca cat gcc aag acg ccg atg tgc ccg gag atc gcg atc gat ctg att       240
Pro His Ala Lys Thr Pro Met Cys Pro Glu Ile Ala Ile Asp Leu Ile
65                  70                  75                  80

gaa gcc ggt gcc tgg ggc gcg acg gtc gcc gat ctc ttc cag gcg gaa       288
Glu Ala Gly Ala Trp Gly Ala Thr Val Ala Asp Leu Phe Gln Ala Glu
                85                  90                  95

gtc ctg ctc aag gcc ggc gtg tcg aac ata ttg atc gcc aac cag atc       336
Val Leu Leu Lys Ala Gly Val Ser Asn Ile Leu Ile Ala Asn Gln Ile
            100                 105                 110

ggc gga ttg aca tcc gcc aga cgc cta cgc atg ctc gca gat gct ttt       384
Gly Gly Leu Thr Ser Ala Arg Arg Leu Arg Met Leu Ala Asp Ala Phe
        115                 120                 125

ccg aaa gcc gag att atc tgc tgt gtc gat tct gtt cag gcc tcg gcc       432
Pro Lys Ala Glu Ile Ile Cys Cys Val Asp Ser Val Gln Ala Ser Ala
    130                 135                 140

aat ctg gtt cag gcc ttt caa ggg cgt gtg gat gcc cca ttc aag gtc 480
Asn Leu Val Gln Ala Phe Gln Gly Arg Val Asp Ala Pro Phe Lys Val
145                 150                 155                 160

ttc atc gaa gtc ggt gtc ggc cgc act ggc gcc cgt acg ttg aat gtt 528
Phe Ile Glu Val Gly Val Gly Arg Thr Gly Ala Arg Thr Leu Asn Val
                165                 170                 175

gca aag gat atc atc gac acc atc tcg aca agt gca gaa atc gta ctg 576
Ala Lys Asp Ile Ile Asp Thr Ile Ser Thr Ser Ala Glu Ile Val Leu
            180                 185                 190

gcc ggt gtg tcg acc tat gaa ggc tcc gtc tcc ggg gaa acg tcg gaa 624
Ala Gly Val Ser Thr Tyr Glu Gly Ser Val Ser Gly Glu Thr Ser Glu
        195                 200                 205

gca ctc gat gca aac atg gcg gcc ctg ttc gat ctc ctg acc gac agt 672
Ala Leu Asp Ala Asn Met Ala Ala Leu Phe Asp Leu Leu Thr Asp Ser
    210                 215                 220

ctt gca tcg ata cgc gaa aaa gat ccc ggg cgc ccg cta acg gtt tca 720
Leu Ala Ser Ile Arg Glu Lys Asp Pro Gly Arg Pro Leu Thr Val Ser
225                 230                 235                 240

gcc ggc ggt tcg atc cat ttc gac cgc gtg ctc gcg gcg ctt gtg ccc 768
Ala Gly Gly Ser Ile His Phe Asp Arg Val Leu Ala Ala Leu Val Pro
                245                 250                 255

gtt tgc gag gcg gat ggc aat gcg acg ttg ttg ctg cgc agc ggc gcc 816
Val Cys Glu Ala Asp Gly Asn Ala Thr Leu Leu Leu Arg Ser Gly Ala
            260                 265                 270

atc ttc ttc tct gat cac ggt gta tat cag cgc ggt ttc cag gca gtc 864
Ile Phe Phe Ser Asp His Gly Val Tyr Gln Arg Gly Phe Gln Ala Val
        275                 280                 285

gac gcc cgc aac cta ctc gca tcc ggc aag gtt gtc ttc aag gca tcc 912
Asp Ala Arg Asn Leu Leu Ala Ser Gly Lys Val Val Phe Lys Ala Ser
    290                 295                 300

gag gca ttt cag ccc tca atg cga atc tgg gcg gag gtc atc tcc gtt 960
Glu Ala Phe Gln Pro Ser Met Arg Ile Trp Ala Glu Val Ile Ser Val
305                 310                 315                 320

cct gag ccg ggg ctg gcg atc gtc ggc atg ggc atg cgg gat gta tcg 1008
Pro Glu Pro Gly Leu Ala Ile Val Gly Met Gly Met Arg Asp Val Ser
                325                 330                 335

ttc gat cag gac ctg ccc gtg gcg ctt cgg ctc cat agg gac gga cat 1056
Phe Asp Gln Asp Leu Pro Val Ala Leu Arg Leu His Arg Asp Gly His
            340                 345                 350

ctg gtc gaa gct gat ctc tct tca tcc gcg aag gtc ggc aag ctc aat 1104
Leu Val Glu Ala Asp Leu Ser Ser Ser Ala Lys Val Gly Lys Leu Asn
        355                 360                 365

gac cag cat gcc ttc ttg tcc ttc ggg aac ggc agc agt ctg gca atc 1152
Asp Gln His Ala Phe Leu Ser Phe Gly Asn Gly Ser Ser Leu Ala Ile
    370                 375                 380

ggc gat gtc ata gaa ttc ggc atc tcg cat ccc tgc acg tgc ttc gat 1200
Gly Asp Val Ile Glu Phe Gly Ile Ser His Pro Cys Thr Cys Phe Asp
385                 390                 395                 400

cgc tgg cgc gtc ttt cac gga atc gat gga tca ggc cgg atc cag cgc 1248
Arg Trp Arg Val Phe His Gly Ile Asp Gly Ser Gly Arg Ile Gln Arg
                405                 410                 415

atc tac aca acc ttc ttt cac tag 1272
Ile Tyr Thr Thr Phe Phe His

            420

<210>  4

<211>  423

<212>  PRT

<213>  Agrobacterium tumefaciens


<400>  4

Met Gln Ser Ser Ser Ala Leu Arg Gln Ser Thr Gly Asp Gln Ser Glu
1               5                   10                  15

Tyr His Ala Gln Ser Asn Met Ile Gly Ser Ser Pro Ala Asp Gly Leu
            20                  25                  30

Leu Ala Leu Pro Leu Leu Thr Val Asp Leu Ala Val Tyr Arg Gly Asn
        35                  40                  45

Arg Asp Arg Phe Leu Ala Leu Val Ser Ala His Gly Ala Lys Ala Ala
    50                  55                  60

Pro His Ala Lys Thr Pro Met Cys Pro Glu Ile Ala Ile Asp Leu Ile
65                  70                  75                  80

Glu Ala Gly Ala Trp Gly Ala Thr Val Ala Asp Leu Phe Gln Ala Glu
                85                  90                  95

Val Leu Leu Lys Ala Gly Val Ser Asn Ile Leu Ile Ala Asn Gln Ile
            100                 105                 110

Gly Gly Leu Thr Ser Ala Arg Arg Leu Arg Met Leu Ala Asp Ala Phe
        115                 120                 125

Pro Lys Ala Glu Ile Ile Cys Cys Val Asp Ser Val Gln Ala Ser Ala
    130                 135                 140

Asn Leu Val Gln Ala Phe Gln Gly Arg Val Asp Ala Pro Phe Lys Val
145                 150                 155                 160

Phe Ile Glu Val Gly Val Gly Arg Thr Gly Ala Arg Thr Leu Asn Val
                165                 170                 175

Ala Lys Asp Ile Ile Asp Thr Ile Ser Thr Ser Ala Glu Ile Val Leu
            180                 185                 190

Ala Gly Val Ser Thr Tyr Glu Gly Ser Val Ser Gly Glu Thr Ser Glu
        195                 200                 205

Ala Leu Asp Ala Asn Met Ala Ala Leu Phe Asp Leu Leu Thr Asp Ser
    210                 215                 220

Leu Ala Ser Ile Arg Glu Lys Asp Pro Gly Arg Pro Leu Thr Val Ser
225                 230                 235                 240

Ala Gly Gly Ser Ile His Phe Asp Arg Val Leu Ala Ala Leu Val Pro
                245                 250                 255

Val Cys Glu Ala Asp Gly Asn Ala Thr Leu Leu Leu Arg Ser Gly Ala
            260                 265                 270

Ile Phe Phe Ser Asp His Gly Val Tyr Gln Arg Gly Phe Gln Ala Val
        275                 280                 285

Asp Ala Arg Asn Leu Leu Ala Ser Gly Lys Val Val Phe Lys Ala Ser
    290                 295                 300

Glu Ala Phe Gln Pro Ser Met Arg Ile Trp Ala Glu Val Ile Ser Val
305                 310                 315                 320

Pro Glu Pro Gly Leu Ala Ile Val Gly Met Gly Met Arg Asp Val Ser
                325                 330                 335

Phe Asp Gln Asp Leu Pro Val Ala Leu Arg Leu His Arg Asp Gly His
            340                 345                 350

Leu Val Glu Ala Asp Leu Ser Ser Ser Ala Lys Val Gly Lys Leu Asn
        355                 360                 365

Asp Gln His Ala Phe Leu Ser Phe Gly Asn Gly Ser Ser Leu Ala Ile
    370                 375                 380

Gly Asp Val Ile Glu Phe Gly Ile Ser His Pro Cys Thr Cys Phe Asp
385                 390                 395                 400

Arg Trp Arg Val Phe His Gly Ile Asp Gly Ser Gly Arg Ile Gln Arg
                405                 410                 415

Ile Tyr Thr Thr Phe Phe His
            420

<210>  5

<211>  32

<212>  DNA

<213>  artificial sequence


<220>

<223>  oligonucleotide primer

<400>  5
cccttaatta atgacgacat ctgataatct tc                                32

<210> 6

<211> 30

<212> DNA

<213> artificial sequence

<220>

<223> oligonucleotide primer

<400> 6
tttgcggccg cttagtggtt atcgcgcggc 30

<210> 7

<211> 31

<212> DNA

<213> artificial sequence

<220>

<223> oligonucleotide primer

<400> 7
cccggtacca tgacgacatc tgataatctt c 31

<210> 8

<211> 32

<212> DNA

<213> artificial sequence

<220>

<223> oligonucleotide primer

<400> 8
cccttaatta atgcagtctt cttcagctct tc 32

<210> 9

<211> 35

<212> DNA

<213> artificial sequence

<220>

<223> oligonucleotide primer

<400> 9

tttgcggccg cctagtgaaa gaaggttgtg tagat                                35

<210>  10

<211>  34

<212>  DNA

<213>  artificial sequence

<220>

<223>  oligonucleotide primer

<400>  10
aaatcatgac tatgcagtct tcttcagctc ttcg                                 34

<210>  11

<211>  33

<212>  DNA

<213>  artificial sequence

<220>
<223>  oligonucleotide primer

<400>  11
tatagatctc tagtgaaaga aggttgtgta gat                                  33